# EUROPÄISCHE PATENTANMELDUNG

(11) **EP 4 379 736 A1**
(43) Veröffentlichungstag der Anmeldung: **05.06.2024**
(21) Anmeldenummer: 22210843.3
(22) Anmeldetag: 01.12.2022
(51) Int. Cl.: G16H 50/20, A61C 13/00, G16H 50/50

(54) **FUNKTIONSABFORMUNG FÜR DENTALPROTHESEN**

(71) Anmelder: Ivoclar Vivadent AG, 9494 Schaan (LI)
(72) Erfinder: Senti, Theresa Sujata Maria, 9497 (LI)
(74) Vertreter: Baldus, Oliver

(57) **Zusammenfassung**

Die vorliegende Erfindung betrifft ein Verfahren zum Bestimmen der dreidimensionalen Form einer Funktionsfläche einer Dentalprothese, mit den Schritten eines Erfassens (S101) eines Intraoralraumes zum Erzeugen eines dreidimensionalen Ist-Datensatzes; und eines Eingebens (S102) des dreidimensionalen Datensatzes in einen angelernten Algorithmus zum Erzeugen eines dreidimensionalen Soll-Datensatzes für eine Funktionsfläche der Dentalprothese.

## Beschreibung

Die vorliegende Erfindung betrifft ein Verfahren zum Bestimmen der dreidimensionalen Form einer Funktionsfläche einer Dentalprothese und eine Vorrichtung zum Bestimmen der dreidimensionalen Form einer Funktionsfläche einer Dentalprothese.

Gegenwärtig werden Dentalprothesen mittels mehrerer Abdruckverfahren hergestellt und anschließend von einem Zahntechniker in eine Dentalprothese umgesetzt werden. Aktuell wird die anatomische Abformung mit einem Standardlöffel und Abdruckmasse erstellt. Dieser Abdruck wird anschließend in ein zahntechnisches Labor geschickt. Dort wird auf Basis dieses Abdrucks der individuelle Löffel für die Funktionsabformung hergestellt.

Dieser Löffel wird anschließend zurück zum Zahnarzt geschickt, der nun die Funktionsabformung für die Dentalprothese durchführen kann. Bei der Funktionsabformung werden neben dem Funktionsrand die Ästhetiklinien und die Okklusionsebene erfasst. Mittels der Dicke der Abformung im anterioren Bereich wird auch die Lippenstütze (Weichgewebsunterstützung/Ästhetik) definiert. Zudem werden auch die Zahnfarbe, die Zahnform oder der Zahntyp bestimmt. Der Abdruck und diese Zusatzinformationen werden schließend dem Zahntechniker übergeben, der nun mit der Herstellung der Totalprothese beginnt. Bisher ist dieses Verfahren analog und es gibt gerade für den zahnlosen Kiefer keine Alternative zu der konventionellen Funktionsabformung mit einem Funktionslöffel.

Es ist die technische Aufgabe der vorliegenden Erfindung, die Gestaltung von Funktionsflächen einer Dentalprothese zu vereinfachen und zu beschleunigen.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zum Bestimmen der dreidimensionalen Form einer Funktionsfläche einer Dentalprothese gelöst, mit den Schritten eines Scannens eines Intraoralraumes zum Erzeugen eines dreidimensionalen Ist-Datensatzes; und eines Eingebens des dreidimensionalen Datensatzes in einen angelernten Algorithmus zum Erzeugen eines dreidimensionalen Soll-Datensatzes für eine Funktionsfläche der Dentalprothese. Zusätzlich können ausgewählte Landmarks sowie ergänzende Datensätze eingegeben werden, wie beispielsweise zweidimensionale Bilder. Bei der Dentalprothese kann es sich um eine Voll- oder eine Teilprothese handeln. Durch das Verfahren kann eine Linie, wie beispielsweise ein Funktionsrand, oder entsprechende Markierungen auf dem dreidimensionalen Datensatz bestimmt werden. Zudem wird eine schnellere, einfachere, und effizientere Herstellung einer Dentalprothese erreicht.

In einer technisch vorteilhaften Ausführungsform des Verfahrens umfasst der angelernte Algorithmus ein künstliches neuronales Netz. Das künstliche neuronale Netz kann ein Deep Neural Network (DNN) oder ein Transformer-Netzwerk sein. Dadurch wird der technische Vorteil erreicht, dass der Soll-Datensatz effizient berechnet werden kann.

In einer weiteren technisch vorteilhaften Ausführungsform des Verfahrens umfasst das künstliche neuronale Netz mehrere Schichten.

In einer weiteren technisch vorteilhaften Ausführungsform des Verfahrens umfasst das künstliche neuronale Netz eine Convolution-Schicht, eine Pooling-Schicht und/oder eine Dense-Schicht.

In einer weiteren technisch vorteilhaften Ausführungsform des Verfahrens wird der Intraoralraum mit einem Scanner gescannt. Im Allgemeinen kann der Intraoralraum durch jedes System zur Erfassung von Daten gescannt werden, die direkt oder indirekt in ein 3D-Modell umgerechnet werden können. Dadurch wird ebenfalls der technische Vorteil erreicht, dass der dreidimensionale Ist-Datensatz schneller als bei einem Abformungsscan erhalten werden kann.

In einer weiteren technisch vorteilhaften Ausführungsform des Verfahrens ist die Funktionsfläche eine Kontaktfläche der Dentalprothese zum Weichteilgewebe. Dadurch wird ebenfalls der technische Vorteil erreicht, dass die Dentalprothese an das Weichteilgewebe angepasst werden kann.

In einer weiteren technisch vorteilhaften Ausführungsform des Verfahrens umfasst der dreidimensionale Ist-Datensatz zusätzliche Daten über eine Weichteilelastizität. Dadurch wird ebenfalls der technische Vorteil erreicht, dass die räumliche Form der Dentalprothese so angepasst werden kann, dass ein Tragekomfort verbessert wird.

In einer weiteren technisch vorteilhaften Ausführungsform des Verfahrens umfasst der dreidimensionale Ist-Datensatz zusätzliche räumliche Daten über einen Oberkiefer, einen Unterkiefer und/oder ein Gesicht. Dadurch wird ebenfalls der technische Vorteil erreicht, dass der Sitz der Dentalprothese im Intraoralraum verbessert wird. Zudem kann zusammen mit den Informationen über ein Gesicht eine Ästhetik besser berücksichtigt oder geplant werden.

In einer weiteren technisch vorteilhaften Ausführungsform des Verfahrens berechnet der angelernte Algorithmus einen Funktionsrand der Dentalprothese. Dadurch wird ebenfalls der technische Vorteil erreicht, dass die Dentalprothese an die funktionellen Bewegungen des Weichgewebes angepasst werden kann.

In einer weiteren technisch vorteilhaften Ausführungsform des Verfahrens berechnet der angelernte Algorithmus eine Zahnaufstellung oder Zahngarnitur. Dadurch wird ebenfalls der technische Vorteil erreicht, dass die Gestaltung der Dentalprothese beschleunigt wird.

In einer weiteren technisch vorteilhaften Ausführungsform des Verfahrens wird die Dentalprothese mit der erzeugten Funktionsfläche hergestellt. Dadurch wird ebenfalls der technische Vorteil erreicht, dass die Dentalprothese automatisch mit der korrekten Funktionsfläche hergestellt werden kann.

Gemäß einem zweiten Aspekt wird die technische Aufgabe durch System zum Bestimmen einer dreidimensionalen Form einer Funktionsfläche einer Dentalprothese gelöst; mit einem Intraoralscanner zum Scannen eines Intraoralraumes zum Erzeugen eines dreidimensionalen Ist-Datensatzes; und einem angelernten Algorithmus zum Eingeben des dreidimensionalen Datensatzes und Ausgeben eines dreidimensionalen Soll-Datensatzes für eine Funktionsfläche der Dentalprothese. Durch das System werden die gleichen technischen Vorteile wie durch das Verfahren nach dem ersten Aspekt erreicht.

In einer technisch vorteilhaften Ausführungsform des Verfahrens umfasst der angelernte Algorithmus ein künstliches neuronales Netz. Dadurch wird der technische Vorteil erreicht, dass der Soll-Datensatz effizient berechnet werden kann.

In einer weiteren technisch vorteilhaften Ausführungsform des Systems umfasst das System eine Herstellungseinrichtung zum Herstellen der Dentalprothese mit der erzeugten Funktionsfläche. Dadurch wird ebenfalls der technische Vorteil erreicht, dass die Dentalprothese automatisch mit der korrekten Funktionsfläche hergestellt werden kann.

Gemäß einem dritten Aspekt wird die technische Aufgabe durch Computerprogramm mit Befehlen gelöst, die bei der Ausführung durch ein System zum Bestimmen einer dreidimensionalen Form einer Funktionsfläche einer Dentalprothese bewirken, dass das Verfahren nach dem ersten Aspekt ausgeführt wird. Durch das Computerprogramm werden die gleichen technischen Vorteile wie durch das Verfahren nach dem ersten Aspekt erreicht.

Ausführungsbeispiele der Erfindung sind in den Zeichnungen dargestellt und werden im Folgenden näher beschrieben.

### Es zeigen:

- Fig. 1: eine schematische Ansicht eines Systems zum Bestimmen einer dreidimensionalen Form einer Funktionsfläche einer Dentalprothese; und
- Fig. 2: ein Blockdiagramm des Verfahrens zum Bestimmen der dreidimensionalen Form einer Funktionsfläche einer Dentalprothese.

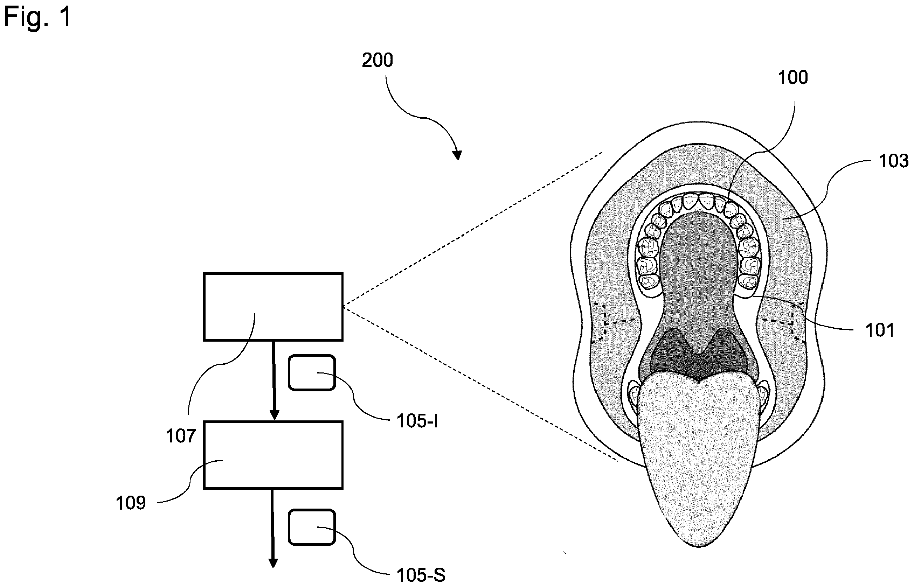

Fig. 1 zeigt eine schematische Ansicht eines Systems 200 zum Bestimmen einer dreidimensionalen Form einer Funktionsfläche 101 einer Dentalprothese 100.

Die Funktionsfläche 101 umfasst beispielsweise das gesamte dreidimensionale Design der Dentalprothese 100. Dabei können anatomische, funktionale und ästhetische Aspekte berücksichtigt werden. Die Funktionsfläche 101 umfasst beispielsweise eine Kontaktfläche der Dentalprothese 100 zum Weichteilgewebe im Mundraum. Die Funktionsfläche 101 umfasst beispielsweise eine Kaufläche oder Zahnaufstellung eines oder mehrerer Zähne. Die Art wie die Dentalprothese 100 ausgestaltet ist, wie beispielsweise durch Form, Größe und/oder Winkel der Zähne, hat einen großen Einfluss auf das Erscheinungsbild der Person, die diese Dentalprothese 100 trägt.

Das System 200 umfasst einen Intraoralscanner 107 zum Scannen eines Intraoralraumes 103, um einen dreidimensionalen Ist-Datensatzes 105-I des Intraoralraumes 103 zu erhalten. Der Ist-Datensatz 105-I gibt die räumliche Form des Weichteilgewebes im Mund an. Zudem können Ober- und Unterkiefer oder Gesicht erfasst werden, um weitere dreidimensionale Daten zu erhalten. Aus den zusätzlichen Scans kann eine Relation zwischen den Scans von Ober- und Unterkiefer und dem Gesicht erhalten werden. Dabei sind die einzelnen Aufnahmen (intraoral und extraoral) lagerichtig präzise zueinander ausgerichtet. Dies ist ein technischer Vorteil zu aktuellen Systemen, bei denen ein Referenzobjekt für ein Best-Fit-Alignement zum Einsatz kommt.

Zudem lassen sich Informationen darüber erfassen, wie die räumliche Lage zwischen den durchgeführten Scans unterschiedlicher Bereiche ist. Des Weiteren können verschiedene Gesichtsausdrücke wie beispielsweise beim Lachen oder Sprechen erfasst werden. Die dreidimensionalen Informationen können aus verschiedenen Blickwinkeln erfasst werden. Im Intraoralscan können weitere Daten, wie beispielsweise ein Funktionsrand, eine Mittellinie, oder eine Campersche-Ebene, angegeben werden.

Zudem können Informationen über das Gesichtsgewebe und die Elastizität des Weichteilgewebes erfasst werden, wie beispielsweise durch eine Magnetresonanzuntersuchung (Magnetic Resonance Imaging - MRI) oder indem das Gesicht und die Mundhöhle gleichzeitig erfasst, gescannt oder gefilmt werden, während der Patient das Gesicht bewegt. Dies kann durch einen Intraoralscanner 107 geschehen, der über einen Luftballon das Gewebe kontrolliert bewegen kann und den Gewebewiderstand direkt ermitteln kann.

Das System umfasst einen angelernten Algorithmus 109 zum Eingeben des dreidimensionalen Ist-Datensatzes 105-I und Ausgeben eines dreidimensionalen Soll-Datensatzes 105-S für eine Funktionsfläche 101 der Dentalprothese 100. Der angelernte Algorithmus 109 wird beispielsweise in einem digitalen Speicher gespeichert und durch einen Prozessor ausgeführt. In dem digitalen Speicher können beispielsweise weitere Daten gespeichert werden, die bei der Ausführung des Algorithmus verwendet werden.

Der angelernte Algorithmus 109 berechnet aus der räumlichen Form des Intraoralraumes 103 automatisch die räumliche Form der Funktionsfläche 101 oder des Funktionsrandes. Hierzu wird der Algorithmus 109 zuvor mit einer Vielzahl von Trainingsdaten angelernt, die eine dreidimensionale Form des Intraoralraumes und eine zugehörige räumliche Form einer Funktionsfläche 101 miteinander verbinden. Der angelernte Algorithmus kann beispielsweise ein künstliches neuronales Netz umfassen. Das künstliche neuronale Netze basiert auf der Vernetzung vieler Neuronen, die jeweils mittels einer Gewichtung miteinander verbunden sind. Das neuronale Netz umfasst mehrere Schichten (Layer), die verschiedene Berechnungen durchführen, wie beispielsweise ein Convolution-Layer, ein Pooling-Layer oder ein Dense-Layer. Das neuronale Netz kann auch auf einer GAN-Architektur (Generative Adversarial Networks) beruhen.

Als Trainingsdaten für das neuronale Netz werden dreidimensionale Datensätze eines Intraoralraumes verwendet, denen jeweils im Voraus ein entsprechender dreidimensionaler Datensatz für die Funktionsfläche 101 zugeordnet ist. Zusätzlich können weiteren Informationen verwendet werden, wie beispielsweise ein Gesichtsscan. Der dreidimensionale Datensatz des Intraoralraumes für die Trainingsdaten kann beispielsweise aus einer Abformung eines zahnlosen Kiefers mit einer hohen Präzision erhalten werden. Diesem Datensatz wird dann eine Funktionsfläche 101 zugeordnet, wie diese beispielsweise mit einem herkömmlichen Verfahren bestimmt worden ist.

Die Gewichtung zwischen den künstlichen Neuronen des künstlichen Neuronalen Netzes werden in der Trainingsphase derart angepasst, dass bei Eingabe eines Datensatzes für den Intraoralraum der jeweilige Datensatz für die Funktionsfläche 101 ausgegeben wird. Wird nach dem Training des neuronalen Netzes dann ein dreidimensionaler Ist-Datensatz 105-I eines Intraoralraumes 103 aus einem durchgeführten Scan eingegeben, wird durch das neuronale Netz automatisch eine Funktionsfläche 101 der Dentalprothese 100 ausgegeben. Dabei wird zu dem eingegebenen Ist-Datensatz 105-I ein Soll-Datensatz für eine Funktionsfläche 101 ausgegeben, die ähnlich zu der Funktionsfläche 101 entsprechender Trainingsdaten ist.

Die dreidimensionalen Datensätze des Intraoralraumes 103 für das Training des neuronalen Netzes können zudem vorgegebene Informationen umfassen, wie beispielsweise:
- Information über eine Kieferrelation einschließlich einer intervestibulären Distanz, einer Ruheschwebelage, oder einer zentrischen Relation;
- Information über eine Okklusionsebene, beispielsweise eine Campersche Ebene oder eine Frankfurter Horizontale;
- schädelbezogene Informationen, beispielsweise eine Relation von Schädel zu Oberkiefer, einen Gesichtsbogen oder ein mittelwertiges Bonwill-Dreieck;
- Informationen über einen Funktionsrand oder eine Bändchenpassage, beispielsweise eine A-Linie, einen äußeren und inneren Ventilrand oder eine Muco-Gingival-Line;
- Informationen über eine Lippenfülle;
- Informationen über eine Bipupillarlinie;
- Informationen über eine Ästhetiklinie, wie beispielsweise eine Mittellinie, eine Eckzahnlinie, eine Lachlinie, oder eine Lippenschlusslinie;
- Informationen über einen Gotischen Bogen, wie beispielsweise einen Pfeilwinkel- oder einen Stützstiftregistrat;
- Informationen über Zahnfarbe und Form;
- Informationen über eine Breite der Nase als Ausgangspunkt für die Größe der Zähne;
- Informationen über eine Papilla Incisiva oder palanine Raphe; und/oder
- Informationen über einen gotischer Bogen (Pfeilwinkel- oder Stützstiftregistrat)

Die Informationen können direkt oder indirekt ermittelt und zur Verfügung gestellt werden. Diese können als Eingabe für die Erzeugung eines Vorschlages verwendet werden, wie die Prothese aussehen könnte. Zusätzlich können diese Informationen als Basis verwendet werden, um das neuronale Netz zu trainieren. Dies kann verwendet werden, um den Patienten zu beraten und verschiedene Varianten von Zahnformen und -größen zu besprechen. Dazu wird die vorgeschlagene Prothese im Mund zusammen mit den Daten angezeigt, die vom Gesicht erfasst worden sind.

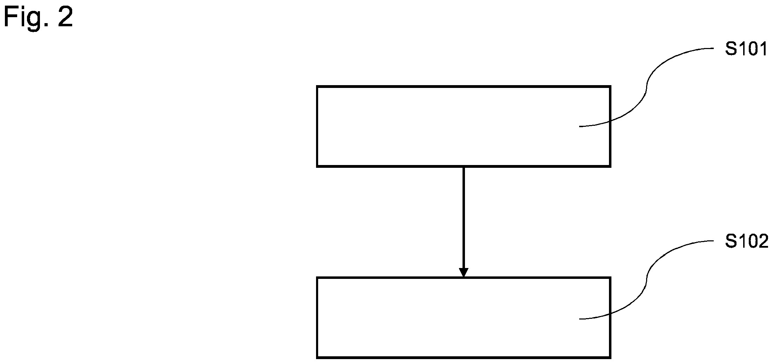

Fig. 2 zeigt ein Verfahren zum Bestimmen der dreidimensionalen Form einer Funktionsfläche 101 einer Dentalprothese 100. Im Schritt S101 wird der Intraoralraum 103 dreidimensional gescannt, um einen dreidimensionalen Ist-Datensatz 105-I zu erhalten. Diese dreidimensionale Ist-Datensatz 105-1 gibt die räumliche Form des Weichteilgewebes im Intraoralraum wieder. Das direkte Erfassen des Weichgewebes kann im Intraoralraum ohne komplexe Geräte und ohne Strahlenbelastung mittels eines Intraoralscanners 107 durchgeführt werden. Beim Scannen kann beispielsweise zusätzlich ein Scan des Ober- oder Unterkiefers einschließlich Weichgewebe durchgeführt werden. Zudem kann ein Scan mit Videos oder Fotos vom Gesicht oder Kopf durchgeführt werden.

In Schritt S102 wird der dreidimensionale Datensatz 105-I in einen angelernten Algorithmus zum Erzeugen eingegeben, um einen dreidimensionalen Soll-Datensatz 105-S für eine Funktionsfläche 101 der Dentalprothese 100 zu erhalten.

Basierend auf dem dreidimensionalen Ist-Datensatz 105-I führt der angelernte Algorithmus eine Berechnung durch, wie die Dentalprothese 100 aussehen soll. Der Benutzer kann anschließend die Ästhetik optimieren. Durch den angelernten Algorithmus können die funktional wichtigen Bereiche der Dentalprothese 100 automatisch bestimmt werden, wie beispielsweise ein Funktionsrand oder Ästhetiklinien. Als Funktionsrand wird der Teil des Prothesenrands bezeichnet, der durch die funktionellen Bewegungen (Mundöffnung, Schlucken, Lippenspitzen) der umgebenden Weichteile bestimmt wird.

Für den Benutzer entsteht eine Zeit- und Kostenersparnis, da keine Modellabdrücke versendet werden müssen. Stattdessen kann die Dentalprothese direkt am Computer gestaltet werden. Dies bedeutet, dass die Dentalprothese mit weniger Arbeitsschritten und in kürzerer Zeit hergestellt werden kann. Das Verfahren implementiert beispielsweise ein generatives Netzwerk zum Vorschlag einer Prothese.

Zudem können die Daten des Eingabedatensatzes 105-1 zusätzlich folgende Informationen umfassen:
- Farbinformationen, wie beispielsweise von dem Weichgewebe oder der Restbezahnung;
- Informationen zum umgebenden Weichgewebe, wie beispielsweise eine Dicke oder Elastizität von Wangen und Lippen;
- Informationen zu einer Restbezahnung, Implantaten oder einer Implantatplanung;
- Informationen einer digitale Volumentomographie (DVT) oder Bildinformationen;
- Informationen zum Gesundheitszustand des Patienten, wie beispielsweise einem Grad der Atrophie oder einer Qualität der Mukosa;
- Informationen über ein Alter, ein Geschlecht, eine Hautfarbe, oder einen Typ, wie beispielsweise kaukasisch, asiatisch;
- Bewegungsinformationen des Unterkiefers; und
- Informationen über einen Transferbogen, wie beispielsweise Fotos oder Videos mit dem Transferbogen im Intraoralraum.

Das Verfahren kann anschließend neben der Ermittlung der Funktionsfläche 101 eine Zahnaufstellung oder Zahngarnitur berechnen. Bei einer Teilprothese kann das Verfahren die Gestaltung der Prothese, Verankerungsvarianten, und/oder Konstruktionsmöglichkeiten der Teilprothese berechnen.

Wenn die Funktionsfläche 101 ermittelt worden ist, kann die Dentalprothese 100 beispielsweise mittels eines Fräsverfahrens aus einem Rohling hergestellt werden. Die anatomischen und ästhetischen Informationen, die zur Herstellung der Dentalprothese benötigt werden, können automatisch aus einer Datenbank abgerufen werden.

Es können zudem definierte Hilfsprothesen verwendet werden, die der Patient temporär einsetzen kann. Diese können in verschiedenen Größen und Formen oder mit auswechselbaren Elementen hergestellt sein, so dass diese individuell anpassbar sind. Dann werden mit jeder Hilfsprothese ein Foto aufgenommen oder ein Scan und eine Sprechprobe durchgeführt. Anschließend wird die optimale Variante bezüglich Ästhetik und Aussprache berechnet. Zudem kann der Zahnarzt Ästhetiklinien temporär auf diese Hilfsprothesen zeichnen, die als weiterer Input für das System dienen.

Das Verfahren kann beispielsweise durch folgenden Code implementiert werden:

```
     path_to_successful_patient_cases =
     "C:\Users\Desktop\SuccessfulPatientCases"
  
     dataset_input = initialize_empty_dataset()
     dataset_expected_output = initialize_empty_dataset()
     n = 0
     for individual_patient_case in
     get_data(path_to_successful_patient_cases):
     patient_n.input.intraoral_scan_data =
     load(individual_patient_case.scandata)
     patient_n.output.functional_margin =
     load(individual_patient_case.functional_margin)
  
     if individual_patient_case.intraoral_images.exists():
     patient_n.input.intraoral_images =
     load(individual_patient_case.intraoral_images)
  
     if individual_patient_case.face_images.exists():
     patient_n.input.face_images =
     load(individual_patient_case.face_images)
  
     if individual_patient_case.face_scan.exists():
     patient_n.input.face_scan =
     load(individual_patient_case.face_scan)
  
     if individual_patient_case.landmarks.exists():
     patient_n.input.landmarks =
     load(individual_patient_case.landmarks)
  
     if individual_patient_case.denture_design.exists():
     patient_n.output.denture_design =
     load(individual_patient_case.denture_design)
  
     if
     individual_patient_case.denture_design_parameter.exists():
     patient_n.output.denture_design_parameter =
     load(individual_patient_case.denture_design_parameter)
  
     dataset_input.add(patient_n.input)
     dataset_expected_output.add(patient_n.output)
     n+= 1
  
     dataset_train = make_dataset(dataset_input,
     dataset_expected_output)
     model = load_model(model_type="GAN")
     compile_options = load_compile_options(model_type="GAN")
  
     model.compile(compile_options)
     model.train(dataset_train, epochs=50)
     model.save('denture_designer_model')
  
     # use model
     model = load('denture_designer_model')
  
     patient_who_needs_a_denture.intraoral_scan_data =
     load(patient_who_needs_a_denture.scandata)
  
     if patient_who_needs_a_denture.intraoral_images.exists():
     patient_who_needs_a_denture.intraoral_images =
     load(patient_who_needs_a_denture_case.intraoral_images)
  
     if patient_who_needs_a_denture.face_images.exists():
     patient_who_needs_a_denture.face_images =
     load(patient_who_needs_a_denture_case.face_images)
  
     if patient_who_needs_a_denture.face_scan.exists():
     patient_who_needs_a_denture.face_scan =
     load(patient_who_needs_a_denture_case.face_scan)
  
     if patient_who_needs_a_denture.landmarks.exists():
     patient_who_needs_a_denture.landmarks =
     load(patient_who_needs_a_denture_case.landmarks)
  
     functional_margin, denture_design,
     denture_design_parameter =
     model.evaluate(patient_who_needs_a_denture)
```

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Dentalprothese
- 101: Funktionsfläche
- 103: Intraoralraum
- 105: Datensatz
- 107: Intraoralscanner
- 109: Algorithmus

- 200: System

## Patentansprüche

1. Verfahren zum Bestimmen der dreidimensionalen Form einer Funktionsfläche (101) einer Dentalprothese (100), mit den Schritten:
- Erfassen (S101) eines Intraoralraumes (103) zum Erzeugen eines dreidimensionalen Ist-Datensatzes (105-I); und
- Eingeben (S102) des dreidimensionalen Datensatzes (105-I) in einen angelernten Algorithmus (109) zum Erzeugen eines dreidimensionalen Soll-Datensatzes (105-S) für eine Funktionsfläche (101) der Dentalprothese (100).

2. Verfahren nach Anspruch 1, wobei der angelernte Algorithmus ein künstliches neuronales Netz umfasst.

3. Verfahren nach Anspruch 2, wobei das künstliche neuronales Netz mehrere Schichten umfasst.

4. Verfahren nach Anspruch 3, wobei das künstliche neuronales Netz eine Convolution-Schicht, eine Pooling-Schicht und/oder eine Dense-Schicht umfasst.

5. Verfahren nach einem der vorangehenden Ansprüche, wobei der Intraoralraum (103) mit einem Scanner gescannt wird.

6. Verfahren nach einem der vorangehenden Ansprüche, wobei die Funktionsfläche (101) eine Kontaktfläche der Dentalprothese (100) zum Weichteilgewebe ist.

7. Verfahren nach einem der vorangehenden Ansprüche, wobei der dreidimensionale Ist-Datensatz (105-I) zusätzliche Daten über eine Weichteilelastizität umfasst.

8. Verfahren nach einem der vorangehenden Ansprüche, wobei der dreidimensionale Ist-Datensatz (105-I) zusätzliche räumliche Daten über einen Oberkiefer, einen Unterkiefer und/oder ein Gesicht umfasst.

9. Verfahren nach einem der vorangehenden Ansprüche, wobei der angelernte Algorithmus (109) einen Funktionsrand der Dentalprothese (100) berechnet.

10. Verfahren nach einem der vorangehenden Ansprüche, wobei der angelernte Algorithmus (109) eine Zahnaufstellung oder Zahngarnitur berechnet.

11. Verfahren nach einem der vorangehenden Ansprüche, wobei die Dentalprothese (100) mit der erzeugten Funktionsfläche (101) hergestellt wird.

12. System zum Bestimmen einer dreidimensionalen Form einer Funktionsfläche (101) einer Dentalprothese; mit:
- einem Intraoralscanner (107) zum Scannen eines Intraoralraumes (103) zum Erzeugen eines dreidimensionalen Ist-Datensatzes (105-I); und
- einem angelernten Algorithmus (109) zum Eingeben des dreidimensionalen Datensatzes (105-I) und Ausgeben eines dreidimensionalen Soll-Datensatzes (105-S) für eine Funktionsfläche (101) der Dentalprothese (100).

13. System nach Anspruch 12, wobei der angelernte Algorithmus ein künstliches neuronales Netz umfasst.

14. System nach Anspruch 12 oder 13, wobei das System eine Herstellungseinrichtung zum Herstellen der Dentalprothese (100) mit der erzeugten Funktionsfläche (101) umfasst.

15. Computerprogramm mit Befehlen, die bei der Ausführung durch ein System zum Bestimmen einer dreidimensionalen Form einer Funktionsfläche (101) einer Dentalprothese bewirken, dass das Verfahren nach einem der Ansprüche 1 bis 11 ausgeführt wird.
